# EUROPÄISCHE PATENTSCHRIFT

(11) **EP 1 722 698 B1**
(45) Veröffentlichungstag und Bekanntmachung des Hinweises auf die Patenterteilung: **07.03.2012**
(21) Anmeldenummer: 05715797.6
(22) Anmeldetag: 07.03.2005
(51) Int. Cl.: A61B 17/34

(54) **VORRICHTUNG ZUR ANSTEUERUNG KÖRPERLICHER STRUKTUREN**
DEVICE FOR CONTROLLING CORPOREAL STRUCTURES
DISPOSITIF POUR COMMANDER DES STRUCTURES CORPORELLES

(30) Priorität: 06.03.2004 DE 202004003646 U
(43) Veröffentlichungstag der Anmeldung: 22.11.2006
(73) Patentinhaber: Medical Intelligence Medizin-Technik GmbH, 86830 Schwabmünchen (DE); ARC SEIBERSDORF RESEARCH GMBH, A-2444 Seibersdorf (AT)
(72) Erfinder: VOGELE, Michael, 86830 Schwabmünchen (DE)
(74) Vertreter: Fiener, Josef
(86) Internationale Anmeldenummer: PCT/EP2005/002386
(87) Internationale Veröffentlichungsnummer: WO 2005/084565

(56) Entgegenhaltungen:
- WO-A-01/34017
- WO-A-97/20515
- WO-A-02/062199
- US-A- 5 281 232
- US-A- 6 106 511
- US-A1- 2004 024 387

## Beschreibung

Die Erfindung betrifft eine Vorrichtung zur Ansteuerung körperlicher Strukturen, insbesondere zur Einführung von Punktionsnadeln oder Operationssonden.

Eine derartige Vorrichtung gemäß dem Oberbegriff des ersten Anspruchs, ist im Grundkonzept aus der WO 97/20515 des Erfinders bekannt, wobei sich diese Zielvorrichtung bei vielen operativen oder stereotaktischen Eingriffen mit genauer Ansteuerung von Punkten am oder im Körper bewährt hat. Vor allem durch die Einbeziehung moderner Computertechnologien wie Computer-Tomographie (CT) ist es möglich geworden, die erforderlichen Eintrittsorte, Eintrittstiefen und Eintrittsrichtungen der medizinischen Instrumente exakt festzulegen, so dass auch eine Zielvorrichtung zur Führung dieser Instrumente der erhöhten Genauigkeit gerecht wird. Mittels der z. B. durch CT ermittelten Patientendaten und Parameter soll dann ein Instrument an den definierten Zielpunkt am oder im Körper gebracht werden können.

Wesentlich bei derartigen Zielvorrichtungen zur Führung medizinischer Instrumente sind eine hohe Zielgenauigkeit und eine schnelle Reproduzierbarkeit. Die weiterhin in der Praxis verwendeten Zielvorrichtungen bestehen meist aus einer massiven Führungsröhre, welche an einen stereotaktischen Rahmen aus Metall-Bügeln oder -Bogen angebracht ist z. B. gemäß der US-A-52 57 998, US-A-51 76 689 oder US-A-52 01 742. Diese Vorrichtungen entsprechen nicht voll den vorstehend genannten Erfordernissen, da diese Zielvorrichtungen mit einem schweren stereotaktischen Rahmens eine reproduzierbare Positionierung sehr schwierig gestalten. Die stereotaktische Genauigkeit leidet bei wiederholten Eingriffen oftmals darunter, da für jeden Patienten die Vorrichtung umgestellt werden muss. Da zudem die herkömmlichen Zielvorrichtungen an einen massiven Rahmen gebunden sind, ist oftmals die Variabilität eingeschränkt. Dies gilt auch für das Ansteuern oder Anfahren der unterschiedlichen Eintrittsorte mit der an diesem Rahmen aufgehängten Zielvorrichtung, insbesondere bei einem stereotaktischen Eingriff, wobei die einzelnen Körperschnitte des Patienten zu einem 3D-Objekt mit entsprechenden stereotaktischen Raumkoordinaten am Beärbeitungscomputer rekonstruiert und auf einen Monitor im Operationssaal transferiert werden. Dieses virtuelle Bild wird im Operationssaal auf den Patienten mit Hilfe eines mit dem Monitor gekoppelten, passiven mechanischen Armes, an dessen Ende sich eine Sonde befindet, geeicht.

Diese Patienteneichung erfolgt durch Anfahren mehrerer Punkte, z. B. anatomisch signifikante Punkte oder durch Röntgen-Eichpunkte (Marker) am Patienten bzw. an der Eichvorrichtung. Nach entsprechender Korrelation zum rekonstruierten 3D-Objekt am Bildschirm ist es dem Computer möglich, dieses 3D-Objekt in diesen virtuellen Raum einzupassen. Der Operateur/Chirurg kann sich während des Eingriffes mit Hilfe von rekonstruiertem 3D-Objekt und mehreren zweidimensionalen Bildern, die immer die Sondenspitze anzeigen, orientieren. Auch bei der Strahlen-Therapie werden Punktionsnadeln (sog. Pins) direkt in das zu bestrahlende Tumorgewebe vorgeschoben und anschließend erfolgt eine direkte Bestrahlung des Tumors durch radioaktive Substanzen, ausgehend von der Nadelspitze. Es gilt in diesem Fall, wenigstens eine Nadel exakt an einen Punkt (z. B. Tumormitte) vorzuschieben und dabei vitale Strukturen zu umgehen und zu schützen. Durch den Einsatz computergestützter Navigationssysteme ist es zwar möglich geworden, entscheidende Verbesserungen auf diesem Gebiet zu erzielen, da auf dem Bildschirm anstatt der Lage der Sondenspitze nun die Lage der Nadelspitze im oder am Körper angezeigt wird. Die Anforderung einer schnellen und einfachen Reproduzierbarkeit bedarf jedoch noch Verbesserungen an der Zielvorrichtung, um die Verstellung in allen drei Raumebenen exakt beibehalten zu können. Selbst für einen geübten Bediener ist dies sehr schwierig, z.B. wegen kleinen, unbewussten Handbewegungen. Aus diesem Grund muss die Nadel oftmals wieder zurückgezogen und korrigiert werden. Sowohl der hohe Zeitaufwand, als auch die Korrektur sind daher patientenbelastend, zumal es oftmals zu minimalen Verbiegungen der extrem dünnen Nadeln kommen kann. Da jedoch Verbiegungen der Nadel vom Computer nicht registriert oder berechnet werden können, liefert der Computer eine Fehlinformation über die momentane Lage der Nadelspitze im Raum, was erhebliche Folgen haben kann.

Demzufolge liegt der Erfindung die Aufgabe zugrunde, eine Vorrichtung zur Ansteuerung körperlicher Strukturen zu schaffen, die vorstehende Anforderungen erfüllt, insbesondere bei einem einfachen Aufbau eine präzise, reproduzierbare und variable Führung für medizinische Instrumente zu schaffen.

Diese Aufgabe wird gelöst durch eine Vorrichtung mit den Merkmalen des Anspruches 1.

Durch die Anbringung von zwei direkt übereinander angeordneten Stellantrieben mit je einem in X-Y-Ebene bewegbaren Verstellarm ist eine beliebige räumliche Positionierung und exakte Ausrichtung der Zieleinrichtung möglich. Dabei sind die Stellantriebe gesondert oder auch wechselweise in ihren X- und Y-Achsen ansteuerbar oder fernbedienbar, so dass durch diese Stellantriebe die Zieleinrichtung exakt und schnell verstellt werden kann, um eine zielgerichtete Einstellung der Zieleinrichtung in den Raumachsen zu ermöglichen. Damit ist eine sehr präzise Anpeilung von vorher beispielsweise im CT festgelegten Eingriffs- oder Zielorten auch von Räumen neben dem Operationssaal möglich, so dass eine wesentliche Reduzierung der Strahlenbelastung des OP-Personals und eine Erleichterung der in der Neurochirurgie üblichen Eingriffe durch die exakte Führung von Instrumenten, insbesondere Punktionsnadeln oder Operationssonden erzielt wird.

Bevorzugte Ausführungsbeispiele sind Gegenstand der Unteransprüche. Zweckmäßig ist hierbei insbesondere die Anordnung der Stellantriebe als flache Boxen übereinander und die zum Patienten hin gekröpfte Gestaltung der Verstellarme, da hierdurch die gewünschten Raumpunkte im Koordinatensystem, ähnlich wie bei den bildgebenden Verfahren als Referenzebenen angefahren werden können. Die Zieleinrichtung weist hierbei insbesondere ein Führungsrohr mit endseitigen Kugelköpfen auf, um die Instrumente möglich weit abstützen zu können. Diese Zieleinrichtung ermöglicht somit das Einführen der Instrumente in exakter Lageeinstellung zueinander. Nachdem die Halterung z. B. etwa auf Tumormitte manuell voreingestellt ist, kann die Feinpositionierung der Instrumente durch die fernsteuerbaren Stellantriebe von einem anderen Raum aus erfolgen, um die Strahlenbelastung des Personals bei einer Strahlentherapie in vorteilhafter Weise zu reduzieren. Nachdem die Zielvorrichtung vorjustiert wurde, kann auch eine Simulation durchgeführt werden, indem z.B. mittels der Stellantriebe die Nadel- oder Sondenspitze im Bereich des stereotaktischen Rahmens umhergeführt wird, wobei auf dem Monitor die Lage im oder am virtuellen Patienten beobachtet werden kann.

Nachfolgend wird ein bevorzugtes Ausführungsbeispiel anhand der Zeichnungen beschrieben. Hierbei zeigen:
Fig. 1 eine Perspektivansicht einer Vorrichtung mit Grundplatte, zwei Basishaltern mit Grund- und Haltestäben, die mit Kugelgelenken verbunden sind;
Fig. 2 eine Perspektivansicht in entgegengesetzter Richtung wie in Fig. 1 mit etwas vergrößerter Darstellung der Zieleinrichtung.

Zur Halterung einer Zieleinrichtung 10 ist eine aus nichtrostendem, magnetisierbarem Stahl gefertigte Grundplatte 1 an ihrer Unterseite mit einem Operationstisch verbunden, beispielsweise mit Metallklauen, die mit der Grundplatte 1 verschraubt sind. Die Grundplatte 1 kann gegenüber dem OP-Tisch in horizontaler und in vertikaler Richtung verstellt werden, wobei eine hohe Festigkeit gewährleistet ist. Die die Zieleinrichtung 10 tragenden Teile der Halterung bestehen jeweils aus einem Basishalter 2, damit gelenkig verbundenen Haltestäben 3 und 4, die am freien Ende jeweils eine Lagerung 5 zur Befestigung von Stellantrieben 6 für die Zieleinrichtung 10 aufweisen. Der Basishalter 2 kann an jeder Stelle der Grundplatte 1 mechanisch, magnetisch oder pneumatisch verankert werden, wobei hierzu ein gerüstartiger Rahmen 1a vorgesehen ist, der den Patienten überspannt. Die gelenkige Verbindung der Haltestäbe 3 und4 ist jeweils mittels Arretierungen, z.B. mit den hier dargestellten Handknebeln fixierbar, so dass die Kraftübertragung und damit die Lagefixierung der Stellantriebe 6 mit ihren in X-Y-Ebene beweglichen Verstellarmen 7 gewährleistet ist.

An den freien Enden der beiden abgekröpften Verstellarme 7 ist ein Führungsrohr 9 für ein medizinisches Instrument 8 zur Ansteuerung eines Zielgewebes Z an Kugelköpfen 9a gelagert. In das Führungsrohr 9 kann eine Sonde oder ein Einlagerohr zur Anpassung an das verwendete Instrument 8 eingebracht werden, insbesondere eine mit einem Anschlag zur Axialeinstellung versehene Punktionsnadel 8, wie dies hier dargestellt ist. Die Axialposition des Instrumentes 8 kann auch durch eine Klemmeinrichtung exakt fixiert werden. Zur Vorjustierung der Zieleinrichtung 10 werden die beiden Basishalter 2 oder der Rahmen 1a auf der Grundplatte 1 mittels Markierungen 1b vorpositioniert, dann z.B. eine Sonde in die Führung 9 eingeführt und anschließend die Sonde so lange im virtuellen Raum herumgeführt, bis die Sondenspitze etwa am gewünschten Eintrittspunkt liegt und die Projektionslinie (= Verlängerung der Sondenspitze entlang der Sondenachse) deckungsgleich mit der Vorschubrichtung ist (welche sich am Bildschirm ablesbar zeigt).

Dann kann das Personal den Bestrahlungsraum verlassen und durch Fernbedienung der Stellantriebe 6 den gewünschte Zielpunkt Z exakt ansteuern. Bei diesem Vorgang wird somit durch Feineinstellung der Verstellarme 7 in X-Y-Ebene der Eintrittspunkt und die Eintrittsrichtung der Zieleinrichtung 10 feinjustiert. Dabei kann vorab durch eine Simulation (ohne Patienten) die gesamte Zieleinrichtung 10 in Relation zum stereotaktischen Rahmen 1 a und damit sowohl zur Eichvorrichtung als auch zum Patienten exakt eingestellt werden. Beim Eingriff wird dann der Patient auf exakt gleiche Art und Weise in den Rahmen 1 a eingepasst, wie er im CT gescannt wurde. Es wird nun das Navigationssystem geeicht und die Zieleinrichtung 10 auf stereotaktisch korrekte Weise angebracht. Zur Kontrolle kann nun nochmals eine Sonde in die bereits justierte Zieleinrichtung 10 eingebracht, und wenn nötig, kann die Sonde nachjustiert werden. Die Nachjustierung ist, falls erforderlich, rasch durchführbar, da die Sonde durch die Vorjustierung schon beinahe die korrekte Lage hat und das Instrument durch die Stellantriebe 6 rasch und exakt auf den Zielort Z nachgestellt werden kann.

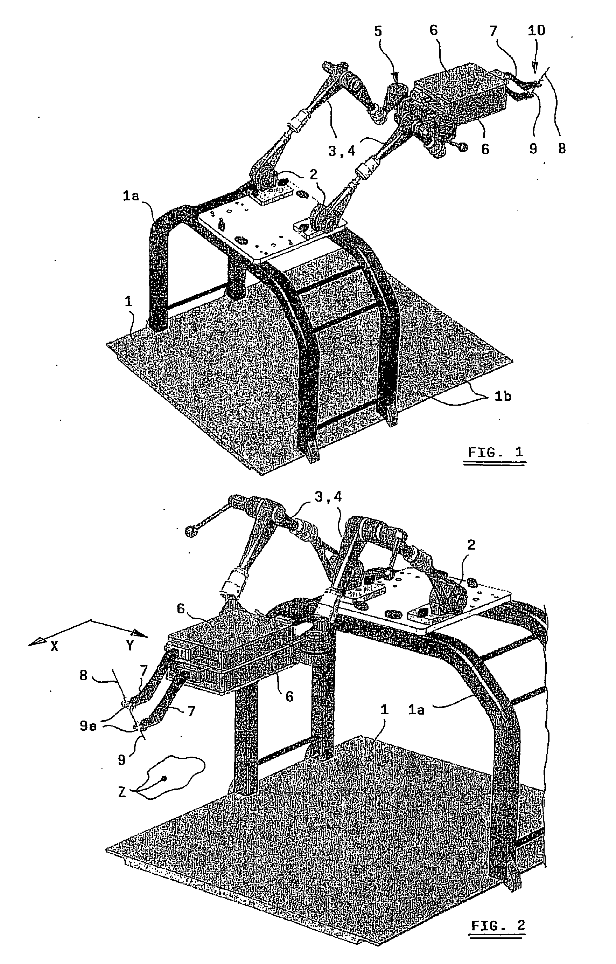

Nach Erreichen des Zielpunktes Z mit der Nadelspitze kann somit die eigentliche Bestrahlung des Tumors beginnen. Nach einmaliger Simulation und Vorjustierung der Sonde kann der Eingriff am Patienten beliebig oft wiederholt werden. Von wesentlicher Bedeutung hierbei ist die Lagerung der Zieleinrichtung 10 mit dem Führungsrohr 9 an den beiden Verstellarmen 7 der übereinander angeordneten Stellantriebe 6. Diese Stellantriebe 6 mit je einem X- und Y-Antriebselement (vorzugsweise Gewindespindeln) in Form eines Kreuzschlittens werden direkt übereinander (vgl. auch Fig. 2) angeordnet. Die Stellantriebe 6 können somit die Verstellarme 7 in Längs- und/oder Querrichtung verfahren, so dass das Führungsrohr 9 in nahezu jede Winkelposition schwenkbar sowie auch verschiebbar angeordnet ist, um die Zieleinrichtung 10 durch die Stellantriebe 6 in X- und/oder Y-Richtung zu verschieben sowie auch um deren Rotationsachsen zu drehen. In Fig. 2 ist die Zieleinrichtung 10 hier nach links oben in die gezeigte Schrägposition geschwenkt. Dabei wird das in das Führungsrohr 9 eingesteckte Instrument 8 ebenfalls mitverschwenkt, so dass von dem ursprünglich angepeilten Ziel Z nunmehr ein anderes Ziel angesteuert werden kann. Hierzu könnte beispielsweise der untere Stellantrieb 6 quer nach links verschoben werden, um das Führungsrohr 9 steiler zu stellen und damit eine millimetergenaue Feineinstellung der Zielrichtung einer relativ dünnen Punktionsnadel zu erreichen.

Im rechten Teil der Fig. 2 ist die Halterung der Stellantriebe 6 näher dargestellt. Diese sind über die Haltestäbe 3 und 4 mit Kugelgelenken mit den Basishaltern 2 verbunden. Die Kugelgelenke sind dabei arretierbar, indem eine Arretierung mittels des Handknebels betätigt wird. Es sei darauf hingewiesen, dass durch die Anordnung der Zieleinrichtung 10 an den beiden Stellantrieben 6 die räumliche Lage des Führungsrohrs 9 zur Führung des Instruments 8 beliebig eingestellt werden kann, wobei eine feinfühlige Verstellung durch die Stellantriebe 6 ermöglicht wird. Somit ist eine Einstellung der Zieleinrichtung 10 in räumlich beliebiger Weise möglich. Auch kann hierbei durch Absenken der Haltestäbe 3 und 4 die Zieleinrichtung 10 zum Patienten hin abgesenkt werden, um möglichst nahe an dem Zielort Z gebracht zu werden und Verbiegungen der dünnen Nadeln zu vermeiden.

## Patentansprüche

1. Vorrichtung zur Ansteuerung körperlicher Strukturen, insbesondere zur Einführung von Punktionsnadeln oder Operationssonden, mit einer Grundplatte (1), wenigstens einem auf der Grundplatte (1) angebrachten Basishalter (2) und daran gelenkig befestigten Haltestäben (3, 4) zur Halterung und Positionierung einer Zieleinrichtung (10) für ein medizinisches Instrument (8), **dadurch gekennzeichnet, dass**
die Zieleinrichtung (10) an zwei Verstellarmen (7) gelagert ist, die jeweils mit einem Stellantrieb (6) am freien Ende der in X- und Y-Ebene mit je einer Lagerung (5) vorpositionierfen Haltestäbe (3, 4) bewegbar sind ,wobei die Grundplatte (1) einen gerüst- oder portalartigen Rahmen (1a) aufweist und die zwei Stellantriebe (6) direkt übereinander angeordnet sind, die vorzugsweise als flache Boxen ausgebildet sind, und zur Feinpositionierung vorgesehen sind.

2. Vorrichtung nach Anspruch 1, **dadurch gekennzeichnet, dass** die Verstellarme (7) zum Patienten hin abgekröpft sind.

3. Vorrichtung nach Anspruch 1 oder 2, **dadurch gekennzeichnet, dass** an den freien Enden der Verstellarme (7) ein Führungsrohr (9) für das medizinische Instrument (8) gelagert ist, insbesondere Ober Kugelköpfe (9a).

4. Vorrichtung nach Anspruch 1, **dadurch gekennzeichnet, dass** die Grundplatte (1) Markierungen (1b) zur Repositionierung des Rahmens (1a) aufweist, der auf der Grundplatte (1) magnetisch, pneumatisch oder mechanisch verankerbar sind.

5. Vorrichturig nach einem der Ansprüche 1 bis 4, **dadurch gekennzeichnet, dass** die Stellantriebe (6) jeweils einen Kreuzschlitten für die Verstellung des jeweiligen Verstellarmes (7) in X-Y-Ebene aufweisen, insbesondere mit fernbedienbaren Gewindespindeln.

## Claims

1. Device for controlling corporeal structures, in particular for introducing puncture needles or surgical probes, comprising a base plate (1), at least one base holder (2) mounted at the base plate (1), and holding rods (3, 4) attached thereto in an articulated manner for holding a targeting device (10) for a medical instrument (8), **characterized in that**
the target device (10) is mounted at two adjustment arms (7) which are each movable by an actuator (6) at the free end of the holding rods (3, 4) being movable in X- and Y-plane and pre-positioned with a bearing (5), each, wherein the base plate (1) comprises a scaffold or portal-like frame (1a) and the two actuators (6) are arranged directly above each other, being preferably formed as flat boxes, and adapted for fine positioning.

2. Device according to claim 1, **characterized in that** the adjustment arms (7) are bent towards the patient.

3. Device according to claim 1 or 2, **characterized in that** a guide tube (9) for the medical instrument (8) is mounted at free ends of the adjustment arms (7), in particular by way of ball heads (9a).

4. Device according to claim 1, **characterized in that** the base plate (1) comprises markings (1 b) for repositioning of the frame (1 a), which can be fastened at the base plate (1) in a magnetic, pneumatic or mechanical manner.

5. Device according to one of claims 1 to 4, **characterized in that** the actuators (6) each comprise a cross-slide for the adjustment of the respective adjusting arm (7) in the X-Y plane, especially with remote-controllable threaded spindles.

## Revendications

1. Dispositif pour commander des structures corporelles, en particulier pour introduire des aiguilles de ponction ou des sondes opératoires, avec une plaque de fond (1), au moins un support de base (2) disposé sur la plaque de fond (1) et des tiges de maintien qui y sont fixées d'une manière articulée (3, 4) en vue du maintien et du positionnement d'un équipement cible (10) pour un instrument médical (8), **caractérisé en ce que** l'équipement cible (10) est logé sur deux bras de réglage (7), qui peuvent être déplacés dans le plan X-Y à chaque fois grâce à un dispositif de positionnement (6) à l'extrémité libre des tiges de maintien (3, 4) pré-positionnées avec un dispositif de logement (5), sachant que la plaque de fond (1) présente un cadre (1a) similaire à une charpente ou un portail et que les deux dispositifs de positionnement (6) sont disposés directement l'un sur l'autre, lesquels dispositifs sont réalisés de préférence sous la forme de boîtes plates, et sont prévues en vue du positionnement fin.

2. Dispositif selon la revendication 1, **caractérisé en ce que** les bras de réglage (7) sont raccourcis en direction du patient.

3. Dispositif selon la revendication 1 ou 2, **caractérisé en ce qu'**aux extrémités libres des arbres de réglage (7) est logé un tube de guidage (9) pour l'instrument médical (8), en particulier par l'intermédiaire de boules sphériques (9a).

4. Dispositif selon la revendication 1, **caractérisé en ce que** la plaque de fond (1) présente des marquages (1b) en vue du repositionnement du cadre (1a), lesquels marquages peuvent être ancrés sur la plaque de fond (1) par voie magnétique, pneumatique ou mécanique.

5. Dispositif selon l'une quelconque des revendications 1 à 4, **caractérisé en ce que** les dispositifs de positionnement (6) présentent chacun dans le plan X-Y un chariot à mouvements croisés pour le déplacement du bras de réglage (7) correspondant, en particulier avec des broches filetées, activables via télécommande.
